# EUROPÄISCHE PATENTSCHRIFT

(11) **EP 2 643 690 B1**
(45) Veröffentlichungstag und Bekanntmachung des Hinweises auf die Patenterteilung: **21.05.2014**
(21) Anmeldenummer: 12711619.2
(22) Anmeldetag: 23.03.2012
(51) Int. Cl.: G01N 33/00, G01N 27/12

(54) **DIODEN-DÜNNSCHICHTANORDNUNG ZUR DETEKTION VON WASSERSTOFF UND VERFAHREN ZU IHRER HERSTELLUNG SOWIE WASSERSTOFFSENSOR**
DIODE THIN FILM ASSEMBLY FOR DETECTING HYDROGEN AND METHOD FOR THE PRODUCTION THEREOF, AND HYDROGEN SENSOR
ENSEMBLE DE COUCHES MINCES DE DIODES POUR LA DÉTECTION D'HYDROGÈNE ET PROCÉDÉ DE PRODUCTION DUDIT ENSEMBLE AINSI QUE DÉTECTEUR D'HYDROGÈNE

(30) Priorität: 25.03.2011 DE 102011015197; 20.05.2011 EP 11166921; 22.12.2011 DE 102011122119
(43) Veröffentlichungstag der Anmeldung: 02.10.2013
(73) Patentinhaber: ODB-Tec GmbH & Co.KG, 41468 Neuss (DE)
(72) Erfinder: OSTERMANN, Dieter, 41468 Neuss (DE); EL ACHHAB, Mhamed, 40217 Düsseldorf (DE); SCHIERBAUM, Klaus, 47803 Krefeld (DE)
(74) Vertreter: Cohausz & Florack
(86) Internationale Anmeldenummer: PCT/EP2012/055189
(87) Internationale Veröffentlichungsnummer: WO 2012/130751

(56) Entgegenhaltungen:
- SHIMIZU ET AL: "H2 sensing performance of anodically oxidized TiO2 thin films equipped with Pd electrode", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 121, Nr. 1, 23. Januar 2007 (2007-01-23), Seiten 219-230, XP005856186, ISSN: 0925-4005, DOI: 10.1016/J.SNB.2006.09.039
- AROUTIOUNIAN ET AL: "Metal oxide hydrogen, oxygen, and carbon monoxide sensors for hydrogen setups and cells", INTERNATIONAL JOURNAL OF HYDROGEN ENERGY, ELSEVIER SCIENCE PUBLISHERS B.V., BARKING, GB, Bd. 32, Nr. 9, 23. Mai 2007 (2007-05-23), Seiten 1145-1158, XP022093237, ISSN: 0360-3199, DOI: 10.1016/J.IJHYDENE.2007.01.004
- WEN-CHAU LIU ET AL: "A new Pt/oxide/In0.49Ga0.51P MOS Schottky diode hydrogen sensor", IEEE ELECTRON DEVICE LETTERS, IEEE SERVICE CENTER, NEW YORK, NY, US, Bd. 23, Nr. 11, 1. November 2002 (2002-11-01), Seiten 640-642, XP011424646, ISSN: 0741-3106, DOI: 10.1109/LED.2002.805006
- YAM ET AL: "Schottky diode based on porous GaN for hydrogen gas sensing application", APPLIED SURFACE SCIENCE, ELSEVIER, AMSTERDAM, NL, Bd. 253, Nr. 24, 15. Oktober 2007 (2007-10-15), Seiten 9525-9528, XP022241164, ISSN: 0169-4332, DOI: 10.1016/J.APSUSC.2007.05.071
- VARGHESE O K ET AL: "Hydrogen sensing using titania nanotubes", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 93, Nr. 1-3, 1. August 2003 (2003-08-01), Seiten 338-344, XP004437120, ISSN: 0925-4005, DOI: 10.1016/S0925-4005(03)00222-3 in der Anmeldung erwähnt
- GARZELLA C ET AL: "TiO2 thin films by a novel sol-gel processing for gas sensor applications", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 68, Nr. 1-3, 25. August 2000 (2000-08-25), Seiten 189-196, XP004216613, ISSN: 0925-4005, DOI: 10.1016/S0925-4005(00)00428-7

## Beschreibung

Die vorliegende Erfindung betrifft einen Wasserstoffsensor mit einer Dioden-Dünnschichtanordnung zur Detektion von Wasserstoff, umfassend eine erste leitfähige Schicht, eine auf die erste leitfähige Schicht aufgebrachte oder auf der ersten leitfähigen Schicht erzeugte Metalloxidschicht und eine auf die Metalloxidschicht aufgebrachte Metallschicht, wobei die Elektronenaustrittsarbeit der ersten leitfähigen Schicht niedriger ist als die Elektronenaustrittsarbeit der Metalloxidschicht, derart, dass zwischen der ersten leitfähigen Schicht und der Metalloxidschicht ein ohmscher Kontakt gebildet ist und wobei zwischen der Metalloxidschicht und der Metallschicht ein Schottky-Kontakt gebildet ist und wobei die Metalloxidschicht eine schwammartig poröse Struktur aufweist und die auf die Metalloxidschicht aufgebrachte Metallschicht eine entsprechende poröse Struktur aufweist. Ferner betrifft die Erfindung ein Verfahren zur Herstellung eines solchen Wasserstoffsensors mit einer Dioden-Dünnschichtanordnung sowie vorteilhafte Verwendungen desselben.

Bei einer Fülle von Anwendungen, insbesondere im Bereich alternativer Antriebskonzepte oder in der Medizin, besteht der Bedarf, die Konzentration von Wasserstoff und anderen Gasen bei unterschiedlichen Umgebungsparametern (Druck, Temperatur,...) präzise und schnell zu bestimmen. Hierzu stehen Gassensoren zur Verfügung, die auf unterschiedlichen Messprinzipien basieren.

Zahlreiche Veröffentlichungen in der wissenschaftlichen und Patentliteratur betreffen Gassensoren, insbesondere Wasserstoffsensoren, die einen Schottky-Kontakt als gassensitives Element aufweisen. So ist in der EP 1 521 080 A1 ein Wasserstoffsensor zur Bestimmung des Wasserstoffgehalts in der Umgebungsatmosphäre beschrieben, welcher eine n-Typ Halbleiterschicht, beispielsweise SiO₂, auf einem Substrat umfasst, auf der wiederum eine dünne semitransparente Metallschicht, beispielsweise Palladium, Platin oder Rhodium, aufgebracht ist, die mit dem n-Typ Halbleitermaterial einen Schottky-Kontakt ausbildet. Die Detektion des Wasserstoffs erfolgt auf optischem Wege in der Weise, dass infolge der Adsorption dissoziierter Wasserstoffatome auf der katalytisch wirkenden Metalloberfläche die Transmission eines eingestrahlten Weißlichts durch die Metallschicht zum Schottky-Kontakt verändert wird, was als Maß für den Wasserstoffgehalt in der Umgebung optisch ausgewertet werden kann.

In der US 2007/0111520 A1 wird ein chemischer Gassensor beschrieben, welcher die Elektron-Loch Produktion an einer Schottky-Barriere zur Detektion von Wasserstoff, Deuterium, Kohlenmonoxid und Sauerstoff nutzt. Der Schottky-Kontakt wird wiederum durch eine Halbleiterschicht, beispielsweise Silizium mit einer (111)-Oberfläche, und eine darauf abgeschiedene sehr dünne Metallschicht (z.B. Au, Ag, Cu, Fe) gebildet.

In der Publikation "Hydrogen sensing using titania nanotubes", Oomman K. Varghese et al. (217 Materials Research Laboratory, The Pennsylvania State University, University Park, PA 16802, USA; Elsevier, Sensors and Actuators B 93 (2003) 338 - 344) wird ein Wasserstoffsensor auf Basis von TiO₂-Nanotubes beschrieben, welche durch Anodisation einer Titanfolie gebildet werden. Die TiO₂-Nanotubes weisen eine Länge von etwa 400 nm und einen Durchmesser von etwa 50 nm auf. Bei einem experimentell vorgenommenen Wechsel zwischen reiner Stickstoffatmosphäre und 1000 ppm Wasserstoff in Stickstoff wird eine Änderung des Widerstands um den Faktor 10³ festgestellt, was als elektrisches Signal für die Wasserstoffkonzentration verwendet werden kann.

In der DE 10 2008 064114 A1 ist ein gasochromer Dünnfilm für einen Wasserstoffsensor hoher Empfindlichkeit beschrieben. Der gasochrome Dünnfilm umfasst ein Übergangsmetalloxid, beispielsweise Wolframoxid, Wolframat, Nioboxid, Nickeloxid oder Titanoxid, bevorzugt Wolframoxid, oder ein Metallhydrid als gasochrome Schicht und eine katalytisch wirkende Schicht, beispielsweise Palladium oder Platin.

In der Veröffentlichung "A study on palladium-titanium oxide Schottky diode as a detector for gaseous components" von Yamamoto et al (Surface Science, Volume 92, Issues 2-3, 2. Feb.1980, pp 400-406) wird schließlich eine durch die Grenzschicht zwischen einem Palladium-Film und einer einkristallinen n-Type Titandioxid-Schicht gebildete Schottky-Barriere beschrieben. Im Einzelnen wird beschrieben, dass ein durch die Schottky-Barriere geleiteter Strom sensitiv auf Wasserstoff oder andere reduzierende Gase in der Umgebung reagiert, wodurch eine solche Schichtanordnung prinzipiell als Gassensor verwendbar ist. Ähnliche elektrische Eigenschaften werden in der Studie an der jeweiligen Grenzfläche zwischen einer TiO₂-Schich und einer Pt-, Au-, Ni-, Al-, Cu-, Mg- oder Zn-Schicht sowie an der jeweiligen Grenzfläche zwischen einer ZnO-, CdS-, GaP- oder einer Si-Schicht und einer Pd-Schicht untersucht. Als TiO₂-Schicht wird ein TiO₂-Einkristall-Wafer mit einer optisch flachen Oberfläche (001) verwendet. Wie die Experimente ferner zeigen, weist die durch eine Palladium-Schicht und eine TiO₂-Schicht gebildete Schottky-Barriere eine bezogen auf Wasserstoff höhere Sensitivität auf als eine jeweils durch Platin oder Gold und TiO₂ gebildete Schottky-Barriere. Die vorstehend beschriebene Dioden-Dünnschichtanordnung zeichnet sich bereits durch einen vergleichsweise einfachen, kostengünstig herzustellenden Aufbau und eine potenziell hohe Zuverlässigkeit aus. Jedoch besitzt sie weiterhin Nachteile hinsichtlich Empfindlichkeit und Robustheit bei wechselnden Umgebungsbedingungen.

Ein gattungsgemäßer Wasserstoffsensor ist weiterhin bekannt aus der Veröffentlichung SHIMIZU ET AL: "H2 sensing performance of anodically oxidized TiO2 thin films equipped with Pd electrode", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 121, Nr. 1, 23. Januar 2007 (2007-01-23), Seiten 219-230.

Weitere im Umfeld der vorliegenden Erfindung zu berücksichtigende Veröffentlichungen sind:
AROUTIOUNIAN ET AL: "Metal oxide hydrogen, oxygen, and carbon monoxide sensors for hydrogen setups and cells", INTERNATIONAL JOURNAL OF HYDROGEN ENERGY, ELSEVIER SCIENCE PUBLISHERS B.V., BARKING, GB, Bd. 32, Nr. 9, 23. Mai 2007 (2007-05-23), Seiten 1145-1158.
WEN-CHAU LIU ET AL: "A new Pt/oxide/In0.49Ga0.51P MOS Schottky diode hydrogen sensor", IEEE ELECTRON DEVICE LETTERS, IEEE SERVICE CENTER, NEW YORK, NY, US, Bd. 23, Nr. 11, 1. November 2002 (2002-11-01), Seiten 640-642.

YAM ET AL: "Schottky diode based on porous GaN for hydrogen gas sensing application", APPLIED SURFACE SCIENCE, ELSEVIER, AMSTERDAM, NL, Bd. 253, Nr. 24, 15. Oktober 2007 (2007-10-15), Seiten 9525-9528.

GARZELLA C ET AL: "TiO2 thin films by a novel sol-gel processing for gas sensor applications", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 68, Nr. 1-3, 25. August 2000 (2000-08-25), Seiten 189-196.

Insgesamt besteht bei sämtlichen aus dem Stand der Technik bekannten Sensoren der Nachteil einer hohen Betriebstemperatur, oftmals bis zu 400°C. Derart hohe Temperaturen stellen in Verbindung mit Wasserstoff ausreichender Konzentration ein entsprechendes Explosionsrisiko dar. Ferner erweist sich bei den vorstehend diskutierten Lösungen der Energieverbrauch insbesondere bei Verwendung von Batterien oder Akkumulatoren als elektrischer Energiequelle als nachteilig. Im Falle der Verwendung elektrochemischer Wasserstoffsensoren ist deren Lebensdauer beziehungsweise deren Verhalten über die Lebensdauer problematisch, da sie üblicherweise einem starken Alterungsprozess unterworfen sind. Außerdem ist die Eignung dieser Wasserstoffsensoren bis in den Temperaturbereich von -40°C nicht gegeben.

Ausgehend liegt der vorliegenden Erfindung die Aufgabe zu Grunde, einen Wasserstoffsensor mit einer Dioden-Dünnschichtanordnung zur Detektion von Wasserstoff anzugeben, welcher sich durch eine gegenüber dem Stand der Technik verbesserte Empfindlichkeit und eine uneingeschränkte Einsetzbarkeit auch bei unterschiedlichen Temperatur- und Druckbedingungen auszeichnet. Ferner soll ein entsprechendes Verfahren zur Herstellung eines solchen Wasserstoffsensors mit einer Dioden-Dünnschichtanordnung sowie vorteilhafte Verwendungen desselben angegeben werden.

Diese und andere Aufgaben werden durch einen Wasserstoffsensor mit einer Dioden-Dünnschichtanordnung zur Detektion von Wasserstoff gemäß dem Patentanspruch 1 gelöst.

Erfindungsgemäß ist zwischen der Metalloxidschicht und der Metallschicht ein gleichrichtender Schottky-Kontakt mit einer nichtlinearen Strom-Spannungs-Kennlinie gebildet. Dieser weist eine sehr hohe Sensitivität für Wasserstoff auf. Mithilfe des erfindungsgemäßen Wasserstoffsensors lässt sich somit Wasserstoff mit sehr hoher Empfindlichkeit und in einem weiten Temperaturbereich bei ungewöhnlich kurzer Ansprechzeit detektieren. Die Querempfindlichkeit des erfindungsgemäßen Wasserstoffsensors bezüglich anderer Gase, wie beispielsweise Kohlenmonoxid oder Stickstoffoxid, erweist sich dabei als außerordentlich gering. Ferner weist die Dioden-Dünnschichtanordnung eine hohe Druckbeständigkeit auf.

Infolge der schwammartig porösen Struktur der erfindungsgemäß in der Dioden-Dünnschichtanordnung vorgesehenen Metalloxidschicht kann das zu detektierende Gas tief in das gesamte Volumen der Schicht eindringen, was bei der kompakten Struktur der aus dem Stand der Technik bekannten Dioden-Dünnschichtanordnungen nicht möglich bzw. bei den teilweise verwendeten TiO₂-Nanotubes auch nur begrenzt möglich ist. Hierdurch wird die Sensitivität der Dioden-Dünnschichtstruktur gegenüber dem zu detektierenden Wasserstoff entscheidend gesteigert und die Ansprechzeit verringert.

Erfindungsgemäß weist die auf die Metalloxidschicht aufgebrachte Metallschicht eine der Metalloxidschicht entsprechende poröse Struktur auf. Dabei ermöglicht die derart stark vergrößerte Oberfläche der Metallschicht eine Dissoziation der H₂-Moleküle, woraufhin die H⁺-Protonen mittels "Spillover"-Effektes zur Metalloxidschicht wandern und dort eine Änderung der elektrischen Eigenschaften der Dioden-Dünnschichtanordnung bewirken. Im Einzelnen verringern die Protonen den Widerstand der TiO₂-Schicht, während die bei der Dissoziation entstehenden Elektronen ins Leitungsband des Platins übergehen und dadurch die Austrittsarbeit des Platins und somit die Schottky-Barriere erniedrigen.

Erfindungsgemäß umfasst die Dioden-Dünnschichtanordnung eine erste leitfähige Schicht, die mit der schwammartig porösen Metalloxidschicht, die auf die erste leitfähige Schicht aufgebracht oder auf der ersten leitfähigen Schicht erzeugt ist, einen ohmschen Kontakt bildet. Die erste leitfähige Schicht übernimmt in der Dioden-Dünnschichtanordnung dabei die Funktion einer Rückelektrode.

Nach einer ersten vorteilhaften Ausgestaltung der Erfindung kann die erste leitfähige Schicht eine Schicht aus Kupfer, Tantal, Titan, Wolfram, Zinn oder einer Legierung hieraus oder eine TCO-Schicht (Transparent Conductive Oxide), insbesondere eine Schicht aus Fluor-dotiertem Zinnoxid (FTO) sein. Erfindungsgemäß entscheidend ist bei der Materialauswahl stets, dass die Austrittsarbeit der ersten leitfähigen Schicht kleiner ist als die Elektronenaffinität der Metalloxidschicht, so dass sich ein ohmscher Kontakt ausbildet. Zwar kann die erste leitfähige Schicht bei Wasserstoffsensoren eine selbsttragende Schicht, beispielsweise eine Folie oder ein Film sein. Erfindungsgemäß ist jedoch vorgesehen, die erste leitfähige Schicht auf einem Substratmaterial, etwa einem Glas- oder Siliziumsubstrat oder auch einem Keramiksubstrat, anzuordnen und demnach nicht selbsttragend auszubilden.

Erfindungsgemäß weist die Metalloxidschicht eine schwammartig poröse Struktur auf. Dabei können die Poren der Metalloxidschicht einen Durchmesser von 50 nm bis 1000 nm, bevorzugt ca. 300 nm, aufweisen. Ferner kann die Dicke der schwammartig porösen Metalloxidschicht 100 nm bis 5 µm, bevorzugt 1µm betragen. Derartige Schichtdicken sind mit üblichen Dünnschichtverfahren problemlos erreichbar.

Die schwammartig poröse Metalloxidschicht kann ferner durch unterschiedliche Metalloxide gebildet sein. Hierbei kommen insbesondere alle Oxide in Betracht, die sich durch Anodisierung der Metalle Kupfer, Tantal, Titan, Wolfram oder Zinn erzeugen lassen. Auch die Mischoxide aus den Legierungen der vorstehend genannten Metalle sind einsetzbar. Besonders bevorzugt ist, dass die schwammartig poröse Metalloxidschicht eine Titanoxid-Schicht (TiOₓ), insbesondere eine TiO₂-Schicht, ist.

Nach einer weiteren vorteilhaften Ausgestaltung der Erfindung ist die erste leitfähige Schicht eine Titanschicht, wobei die schwammartig poröse Metalloxidschicht eine durch Anodisieren der Oberfläche der Titanschicht als erster leitfähiger Schicht gebildete Titanoxidschicht ist. Durch entsprechende Einstellung der Anodisierungsparameter, wie z.B. Anodisierspannung oder Zusammensetzung des Elektrolyten, kann die Struktur der auf der Titanoberfläche erzeugten Titanoxidschicht unmittelbar beeinflusst werden. Entsprechend dem Vorstehenden ist es ebenso möglich, die schwammartig poröse Metalloxidschicht durch Anodisieren der Metalle Kupfer, Tantal, Titan, Wolfram oder Zinn zu erzeugen. Die Anodisierung von Titan ist jedoch bevorzugt.

Ergänzend zur Erzeugung der schwammartig porösen Metalloxidschicht auf der ersten leitfähigen Schicht mittels Anodisierung kann eine schwammartig poröse Metalloxidschicht mittels additivem Sol-Gel-Prozess auf der durch Anodisierung der ersten leitfähigen Schicht erzeugten schwammartig porösen Metalloxidschicht abgeschieden werden. Eine mittels Sol-Gel-Prozess abgeschiedene Metalloxidschicht ist in Vergleich zu einer durch Anodisierung erzeugten Metalloxidschicht stark amorph ausgebildet, was zu einer besonders großen für die Wasserstoffdetektion aktiven Oberfläche führt. Bevorzugt sind die durch Anodisierung erzeugte Metalloxidschicht und die darauf in einem Sol-Gel-Verfahren aufgebrachte Metalloxidschicht chemisch identisch. Sol-Gel-Prozesse aus dem Stand der Technik bekannt und für Dünnschichtverfahren etabliert.

Erfindungsgemäß umfasst die Dioden-Dünnschichtanordnung weiterhin eine auf die schwammartig poröse Metalloxidschicht aufgebrachte Metallschicht, welche eine entsprechende poröse Struktur aufweist und mit der schwammartig porösen Metalloxidschicht einen Schottky-Kontakt ausbildet. Die poröse Metallschicht fungiert bei dem erfindungsgemäßen Wasserstoffsensor somit als Frontelektrode.

Erfindungsgemäß ist zwischen der Metalloxidschicht und der Metallschicht ein Schottky-Kontakt gebildet, d.h. die poröse Metallschicht weist eine höhere Elektronenaustrittsarbeit auf als die Metalloxidschicht. Insbesondere handelt es sich bei der Metallschicht umeine Platin-, Palladium- oder Goldschicht. Bevorzugt weist die poröse Metallschicht dabei eine kristallitische Struktur auf, wobei die Kristallite einen Durchmesser von 5 nm bis 100 nm aufweisen.

Erfindungsgemäß sind die erste leitfähige Schicht und die poröse Metallschicht mit einer elektronischen Ansteuerung-und Auswerteeinheit verbunden.

Erfindungsgemäß sind die erste leitfähige Schicht als Rückelektrode und die poröse Metallschicht als Frontelektrode mit einer elektronischen Ansteuerung- und Auswerteeinheit verbindbar. Dabei kommt der elektronischen Ansteuerung- und Auswerteeinheit einerseits die Funktion der Energieversorgung der Dioden-Dünnschichtanordnung und andererseits die Funktion einer schnellen Auswerteelektronik zu, welche eine Änderung der elektrischen Eigenschaften der Dioden-Dünnschichtanordnung infolge ihrer Beaufschlagung mit Wasserstoff, beispielsweise als Strom- und/oder Spannungsänderung, in ein zur Wasserstoffkonzentration bevorzugt proportionales Signal umsetzt.

Zu den besonderen Vorteilen des erfindungsgemäßen Wasserstoffsensors wird auf das Vorstehende verwiesen.

Nach einer Ausgestaltung der Erfindung sind die erste leitfähige Schicht mit dem Minuspol und die poröse Metallschicht mit dem Pluspol der elektronischen Ansteuerung-und Auswerteeinheit verbindbar, so dass die Dioden-Dünnschichtanordnung in Durchlassrichtung betrieben werden kann. Ein Betrieb der Dioden-Dünnschichtanordnung in Durchlassrichtung hat den Vorteil, dass die Sensor-Funktion in einem besonders weiten Temperaturbereich insbesondere auch weit unterhalb des Gefrierpunktes gewährleistet ist. Umfasst die Dioden-Dünnschichtanordnung des Wasserstoffsensors beispielsweise eine schwammartig poröse Metalloxidschicht in Form einer schwammartig porösen TiO₂-Schich und ist die auf die Metalloxidschicht aufgebrachte Metallschicht eine Platin-oder Palladiumschicht, so kann der Wasserstoffsensor bei einem Betrieb der Dioden-Dünnschichtanordnung in Durchlassrichtung in einem Bereich von >125°C bis unter -40°C betrieben werden.

Alternativ kann die Dioden-Dünnschichtanordnung auch in Sperrrichtung, d.h. mit positiv gepolter Rückelektrode (erste leitfähige Schicht) und negativ gepolter Frontelektrode (poröse Metallschicht), betrieben werden. Hierbei weist die Strom-Spannung-Kennlinie der Dioden-Dünnschichtanordnung jedoch einen weitgehend linearen Verlauf mit gegenüber einem Betrieb in Durchlassrichtung reduzierter Sensitivität für Wasserstoff auf.

Der erfindungsgemäße Wasserstoffsensor kann apparativ und schaltungstechnisch in unterschiedlicher Weise ausgebildet sein. So kann er nach einer Ausgestaltung der Erfindung in ein TO-Gehäuse eingebaut oder Teil eines Leadframe- oder LCC-Packages sein. Dabei kann der Wasserstoffsensor einzeln oder in Form eines Arrays in dem TO-Gehäuse bzw. LCC-Package vorliegen.

Nach einer weitergehenden Ausgestaltung der Erfindung kann das TO-Gehäuse mit einer wasserundurchlässigen und dampfdiffusionsoffenen und/oder gasdurchlässigen Membran, beispielsweise mit einer PTFE-Membran (Gore-Tex® der W. L. Gore & Associates) oder mit einer sogenannten "Pall-Membran", abgedeckt werden, um eine die Sensorfunktion der Dioden-Dünnschichtanordnung beeinträchtigende Beaufschlagung der Dioden-Dünnschichtanordnung mit Wasserdampf zu vermeiden. Bei der Pall-Membran kann es sich beispielsweise um ein modifiziertes Polethersulfon-Polymer auf einem nichtgewebten Polyesterträger (Handelsname: Supor® R) oder um eine acrylische Copolymermembran auf einem dünnen nichtgewebten Polyesterträger (Handelsname: Versapor® TR) handeln. Nach einer besonders bevorzugten Ausgestaltung der Erfindung kann das TO-Gehäuse hierfür auch ein Molekularsieb für Wasserdampf umfassen. Dieses kann in Form von Zeolit-Kügelchen mit 3 Angström Porendurchmesser vorliegen, wodurch Wasserdampf von der Dioden-Dünnschichtanordnung effektiv ferngehalten werden kann. Ferner können die Zeolit-Kügelchen ausheizbar ausgeführt sein. Alternativ oder ergänzend zu den Zeolit-Kügelchen können auch geeignete Gettersysteme verwendet werden, an welchen der störende Wasserdampf adsorbiert wird.

Nach einer weiteren Ausgestaltung der Erfindung ist der Wasserstoffsensor als SMD-Bauteil ausgeführt. Es versteht sich, dass dabei auch eine elektronische Ansteuerung- und Auswerteeinheit in dem SMD-Bauteil integriert sein kann.

Die eingangs genannte Aufgabe wird verfahrensmäßig durch ein Verfahren zur Herstellung eines erfindungsgemäßen Wasserstoffsensors mit einer Dioden-Dünnschichtanordnung gemäß Anspruch 6 gelöst. Dieses Verfahren weist folgende Verfahrensschritte auf:
- Bereitstellen der ersten leitfähigen Schicht,
- Aufbringen der Metalloxidschicht auf die erste leitfähige Schicht oder Erzeugen der Metalloxidschicht auf der ersten leitfähigen Schicht, wobei die Metalloxidschicht derart durch ein elektrochemisches Verfahren oder durch einen Sol-Gel-Prozess aufgebracht oder erzeugt wird, dass sie eine schwammartig poröse Struktur ausbildet,
- Aufbringen der Metallschicht auf Metalloxidschicht, wobei die Metallschicht derart durch ein elektrochemisches Verfahren oder durch ein Vakuumabscheideverfahren auf die Metalloxidschicht aufgebracht wird, dass die auf die Metalloxidschicht aufgebrachte Metallschicht eine der schwammartig porösen Struktur der Metalloxidschicht entsprechende poröse Struktur aufweist.

Das erfindungsgemäße Verfahren kann mit geringem apparativem Aufwand und zu geringen Kosten durchgeführt werden, wodurch Dioden-Dünnschichtanordnungen mit den vorstehend im Detail beschriebenen vorteilhaften Eigenschaften sehr kostengünstig bereitgestellt werden können. Die hohe Umweltverträglichkeit des erfindungsgemäßen Verfahrens ermöglicht eine weitere Senkung der Produktionskosten.

Nach einer Ausgestaltung des erfindungsgemäßen Verfahrens ist die erste leitfähige Schicht eine Titanschicht und die Metalloxidschicht wird durch Anodisierung auf der Titanschicht erzeugt, wobei die Anodisierung der Titanschicht bei einer Anodisierspannung von 90 bis 200 V, bevorzugt ca. 120 V erfolgt. Dabei erfolgt die Anodisierung bevorzugt in 0,5 bis 14 molarer Schwefelsäure, besonders bevorzugt ca. 3,5 molarer Schwefelsäure.

Nach einer weiteren vorteilhaften Ausgestaltung der Erfindung kann die Metalloxidschicht mittels PVD auf die erste leitfähige Schicht aufgebracht werden. Speziell kann im Falle einer Titanschicht als erster leitfähiger Schicht die Titanoxidschicht alternativ oder ergänzend zu einer Anodisierung auch mittels eines PVD-Verfahrens auf der Titanschicht abgeschieden werden.

Nach einer weiteren Ausgestaltung der Erfindung kann auf die durch Anodisierung der ersten leitfähigen Schicht erzeugte schwammartig poröse Metalloxidschicht eine weitere schwammartig poröse Metalloxidschicht durch einen Sol-Gel-Prozess aufgebracht werden. Bevorzugt sind dabei die schwammartig poröse Metalloxidschicht und die weitere schwammartig poröse Metalloxidschicht chemisch identisch. Beispielsweise handelt es sich jeweils um eine TiO₂-Schicht. Die durch den Sol-Gel-Prozess auf die bereits auf der Oberfläche der ersten leitfähigen Schicht erzeugte schwammartig poröse Metalloxidschicht aufgebrachte weitere schwammartig poröse Metalloxidschicht zeichnet sich durch eine besonders amorphe Struktur aus, wodurch sich eine besonders große für die Wasserstoffdetektion aktive Oberfläche erzielen lässt.

Die Metallschicht wiederum kann mittels eines PVD-Verfahrens, insbesondere mittels Sputtern oder Aufdampfen, auf die schwammartig poröse Metalloxidschicht aufgebracht werden. Alternativ kann die Metallschicht auch in einem galvanischen Prozess auf der schwammartig porösen Metalloxidschicht abgeschieden werden. So kann beispielsweise eine Platinschicht galvanisch aus Hexachloridoplatinsäure H₂[PtCl₆] abgeschieden werden.

Nach einer vorteilhaften Ausgestaltung der Erfindung kann schließlich die Metallschicht mit einer Schutzschicht versehen werden, wobei diese bevorzugt eine Tetraethylorthosilikat-, Hexamethyldisiloxan- oder Hexamethyldisilazanschicht ist. Die Schutzschicht hat üblicherweise eine Dicke von lediglich 10 - 50 nm und beeinträchtigt die Gebrauchsfähigkeit der Dioden-Dünnschichtanordnung nicht, da Wasserstoff problemlos durchdiffundieren kann.

Im Folgenden wird die Herstellung einer Dioden-Dünnschichtanordnung exemplarisch und ohne den Erfindungsgegenstand einzuschränken erläutert.

### Beispiel 1

Auf einer selbsttragende Reintitanfolie Grade 1 mit Fremdelementen folgender Zusammensetzung. Feₘₐₓ 0,20%; Cₘₐₓ 0,10%; Nₘₐₓ 0,03%; Hₘₐₓ 0,015%; 0ₘₐₓ 0,18%) oder Grade 2 (Feₘₐₓ 0,20%; Cₘₐₓ 0,10%; Nₘₐₓ 0,03%; Hₘₐₓ 0,015%; Oₘₐₓ 0,25%) der Firma Schumacher Titan mit einer Dicke von 0,25 mm als Rückelektrode wird mittels Anodisation eine schwammartig poröse Titanoxidschicht mit einer Dicke von 100 bis 5000 nm, vorliegend von ca. 1000 nm erzeugt. Dabei wird eine Anodisierspannung von 90 bis 200 V, vorliegend 90 V gewählt. Als Elektrolyt wird 14 bis unter 0,5 molare, vorliegend 3,5 molare, Schwefelsäure eingesetzt. Durch Röntgenbeugungsversuche konnte ermittelt werden, dass bei Spannungen bis etwa 100 Volt eine TiO₂-Anatas-Kristallform erzeugt wird, während bei Spannungen über 110 Volt die gewünschte Rutil-Kristallform mit einer sehr großen aktiven Oberfläche erzeugt wird.

Auf die schwammartig poröse TiO₂-Schich wird mittels Sputtern eine 10 bis 100 nm dicke Platinschicht als Frontelektrode aufgebracht. Alternativ kann die Platinschicht auch galvanisch erzeugt werden, wobei als Elektrolyt Hexachloridplatinsäure H₂[PtCl₆] verwendet wird. Hierdurch wird sichergestellt, dass die Platinschicht eine der schwammartig porösen Struktur der TiO₂-Schich entsprechende poröse Struktur aufweist. Dabei wird die Platinschicht aus Kristalliten mit einem Durchmesser von 5 bis 100 nm gebildet. Die exakte Dicke der Platinschicht wird in dem genannten Bereich so gewählt, dass eine gute ohmsche Kontaktierung möglich ist. Als Kontaktierungsverfahren können Drahtbondverfahren, Reibschweißen oder ein Klebeprozess, der eine leitfähige stoffschlüssige Verbindung ermöglicht, eingesetzt werden.

### Beispiel 2

Im Unterschied zum vorstehenden Beispiel wird die Rückelektrode der Dioden-Dünnschichtanordnung bildende Titanschicht erfindungsgemäß nicht selbsttragend, sondern als auf einem Glassubstrat mittels Sputtertechnik oder als galvanisch abgeschiedene Schicht ausgebildet. Auf dieser Schicht wird analog zu Beispiel 1 eine schwammartig poröse TiO₂-Schich mittels Anodisierung der Titanschicht erzeugt. Die Platinschicht als Frontelektrode wird ebenfalls analog zu Beispiel 1 mittels Sputtern oder auch galvanisch auf der schwammartig porösen TiO₂-Schich abgeschieden.

### Beispiel 3

Auf ein Glassubstrat wird eine elektrisch leitfähige TCO-Schicht (z.B. Fluor-dotiertes Zinnoxid) aufgebracht. Auf diese wird wiederum eine Titanschicht aufgebracht, insbesondere aufgesputtert, an deren Oberfläche mittels Anodisieren eine schwammartig poröse TiO₂-Schicht als Metalloxidschicht erzeugt wird. Der Vorteil der Unterlegung der Titanschicht mit der TCO-Schicht besteht darin, dass zur Anodisierung der Titanschicht der Strom nicht lateral in die Titanschicht eingeleitet werden muss, sondern die gesamte mit der TCO-Schicht kontaktierte Fläche hierfür zur Verfügung steht.

Auf die schwammartig poröse TiO₂-Schich wird in der vorstehend beschriebene Weise die Platinschicht als Frontlelektrode der Dioden-Dünnschichtanordnung aufgebracht.

Ein weiterer Aspekt der vorliegenden Erfindung betrifft die Verwendung des erfindungsgemäßen Wasserstoffsensors in einem mit Wasserstoff betriebenen Fahrzeug oder in Wasserstofftanks und -leitungen zur Erfassung von Leckagen. Zu den Vorteilen einer solchen Verwendung wird auf das Vorstehende verwiesen.

Ein weiterer Aspekt der vorliegenden Erfindung betrifft die Verwendung des erfindungsgemäßen Wasserstoffsensors in einer Sensoranordnung zur Messung der Zusammensetzung von Gasen, insbesondere von Abgasen oder Stadtgas. Stadtgas hat im Unterschied zu Erdgas einen vergleichsweise hohen Wasserstoffanteil, so dass die Vorteile des erfindungsgemäßen Wasserstoffsensors hier voll zum Tragen kommen. Zu den übrigen Vorteilen einer solchen Verwendung wird auf das Vorstehende verwiesen.

Ein weiterer Aspekt der vorliegenden Erfindung betrifft die Verwendung des erfindungsgemäßen Wasserstoffsensors in einer schnellen Feuermeldereinheit. Diesem liegt die Überlegung zugrunde, dass bei einem ausbrechenden Feuer Wasserstoff aufgrund seiner hohen Flüchtigkeit noch vor den Rauchgasen als erstes Gas detektiert werden kann, so dass ein auf dem erfindungsgemäßen Wasserstoffsensor basierender Feuermelder sich durch eine bezogen auf die Brandentstehung besonders kurze Ansprechzeit auszeichnet.

Ein weiterer Aspekt der vorliegenden Erfindung betrifft die Verwendung des erfindungsgemäßen Wasserstoffsensors als UV-Sensor. Der erfindungsgemäße Wasserstoffsensor kann hierbei als selektiver UV-Sensor betrieben werden, wobei sie eine hohe Empfindlichkeit unterhalb einer Wellenlänge < 380 nm, d.h. bereits im nahen UV, aufweist. Bei Bestrahlung mit UV-Licht ist dementsprechend ein signifikanter Anstieg des Stromflusses innerhalb der Dioden-Dünnschichtanordnung feststellbar. Wie der Erfinder in überraschender Weise gefunden hat, ist die Dioden-Dünnschichtanordnung jedoch im Bereich des sichtbaren Lichts unempfindlich ("visible blind"), so dass hier das Potenzial besteht, deutlich teurere UV-Sensoren auf SiC-Basis zu ersetzen.

Ein weiterer Aspekt der vorliegenden Erfindung betrifft die Verwendung des erfindungsgemäßen Wasserstoffsensors als elektrische Energiequelle.

Eine als elektrische Energiequelle ("Chemogenerator") verwendete Dioden-Dünnschichtanordnung kann als Dünnschicht-Einkammer-Festoxid-Brennstoffzelle bezeichnet werden. Im Unterschied zu Standard-Brennstoffzellen müssen die Gase nicht getrennt zugeführt werden. Wenn ein Wasserstoff oberhalb eines gewissen Schwellwerts und Restsauerstoff enthaltender Gasstrom die Dioden-Dünnschichtanordnung umspült, kann an deren Elektroden (Front- und Rückelektrode) ein elektrisches Signal abgegriffen werden, das in einer weiteren Elektronik für Steuerungs- und Alarmfunktionen verwendet werden kann. Es ist auch möglich, einen Wasserstoffgasstrom in Luft auf die Dioden-Dünnschichtanordnung zu leiten, so dass der Sauerstoff aus der Luft kontinuierlich an der TiO₂-Oberfläche adsorbiert wird.

Wie Untersuchungen der Erfinder zeigen, muss Sauerstoff der Dioden-Dünnschichtanordnung nicht kontinuierlich zugeführt werden. Infolge der starken Adsorption von Luftsauerstoff an der Metalloxidschicht ist nach einmaliger Sauerstoffexposition der Dioden-Dünnschichtanordnung auch ein Betrieb der Dioden-Dünnschichtanordnung als elektrische Energiequelle in einem sauerstofffreien Gasstrom für einen begrenzten Zeitraum möglich.

Physikalisch betrachtet läuft an der Oberfläche der porösen Metallschicht (Frontelektrode) zur schwammartig porösen Metalloxidschicht hin eine exergonische chemische Reaktion zwischen Wasserstoff und Sauerstoff ab, wobei zwischen Front-und Rückelektrode der Dioden-Dünnschichtanordnung eine elektromotorische Kraft erzeugt wird, aus der eine Stromdichte >1 mA/cm² bei der Konzentration von > 0,1% Wasserstoff in dem umgebenden beziehungsweise vorbeiströmenden Gas, beispielsweise Luft oder Abluft, resultiert. Für den Wirkungsgrad der durch die Dioden-Dünnschichtanordnung gebildeten elektrischen Energiequelle kommt es wiederum entscheidend auf die schwammartige Porosität der Metalloxidschicht an.

Im Folgenden wird die Erfindung anhand Ausführungsbeispiele darstellender Zeichnungen näher erläutert. Es zeigen:
- Fig. 1: eine Dioden-Dünnschichtanordnung zur Detektion von Wasserstoff in einer schematischen Schnittansicht,
- Fig. 2: einen Wasserstoffsensor mit der Dioden-Dünnschichtanordnung aus Fig. 1 in einer ersten Ausführungsform in perspektivischer Darstellung,
- Fig. 3: einen Wasserstoffsensor mit der Dioden-Dünnschichtanordnung aus Fig. 1 in einer zweiten Ausführungsform in perspektivischer Darstellung,
- Fig. 4: ein Ersatzschaltbild für einen Wasserstoffsensor,
- Fig. 5: den in ein TO-Gehäuse integrierten Wasserstoffsensor der Fig. 2 in schematisierter perspektivischer Darstellung,
- Fig. 6: den in ein TO-Gehäuse integrierten Wasserstoffsensor der Fig. 3 in Form eines SMD-Bauteils in schematisierter perspektivischer Darstellung,
- Fig. 7: den Wasserstoffsensor der Figur 2 als SMD-Bauteil auf einer Platine,
- Fig. 8: eine elektronenmikroskopische Aufnahme einer anodisierten Titanschicht,
- Fig. 9: eine elektronenmikroskopische Aufnahme der anodisierten Titanschicht mit galvanisch aufgebrachter Platinschicht,
- Fig. 10: eine Strom-Spannungskennlinie der Dioden-Dünnschichtanordnung aus Fig. 1,
- Fig. 11: eine Spannungstransiente bei der Umspülung der Dioden-Dünnschichtanordnung aus Fig. 1 in einem Wasserstoff und Sauerstoff enthaltenen Gasstrom,
- Fig. 12: Strom- und Spannungstransienten der Dioden-Dünnschichtanordnung aus Fig. 1 in einem Wasserstoff und Sauerstoff enthaltenen Gasstrom und
- Fig. 13a,b: einen Verlauf des Stroms in Abhängigkeit von der Zeit bei einer Umspülung der Dioden-Dünnschichtanordnung aus Fig. 1 mit Luft, in der Wasserstoff unterschiedlicher Konzentration enthalten ist.

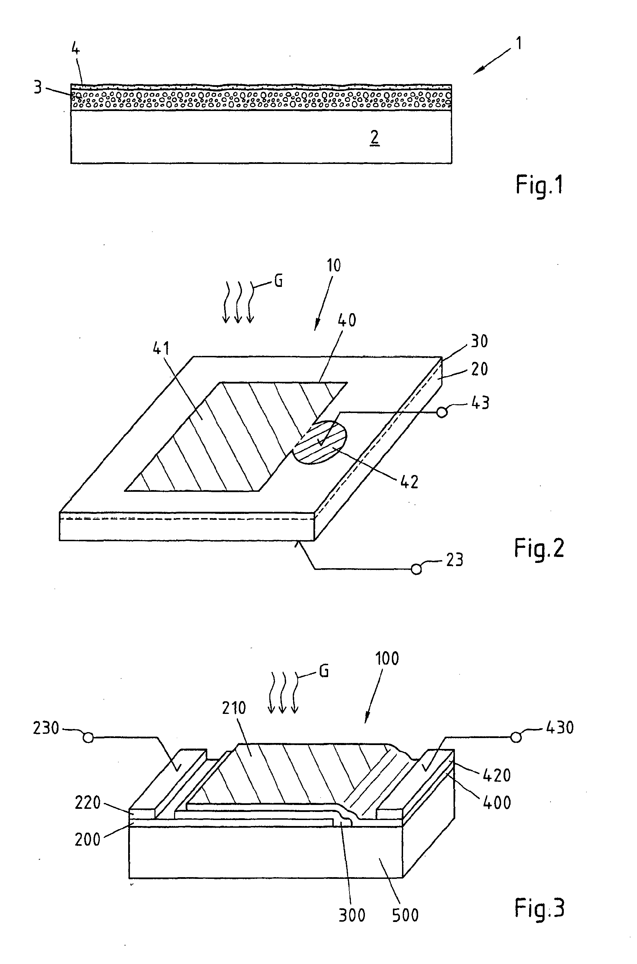

Fig. 1 zeigt eine Dioden-Dünnschichtanordnung 1 zur Detektion von Wasserstoff in einer schematischen Schnittansicht. Die Dioden-Dünnschichtanordnung 1 umfasst eine erste leitfähige Schicht 2 als Rückelektrode und darüber angeordnet eine Metalloxidschicht 3, die ihrerseits von einer Metallschicht 4 als Frontelektrode bedeckt wird. Die Elektronenaustrittsarbeit der ersten leitfähigen Schicht 2 ist niedriger gewählt als die Elektronenaustrittsarbeit der Metalloxidschicht 3, so dass zwischen der ersten leitfähigen Schicht 2 und der Metalloxidschicht 3 ein ohmscher Kontakt gebildet ist.

Die erste leitfähige Schicht 2 kann eine Schicht aus Kupfer, Tantal, Titan, Wolfram, Zinn oder einer Legierung hieraus oder eine TCO-Schicht, insbesondere eine Schicht aus Fluor-dotiertem Zinnoxid ist (FTO) sein. Vorliegend ist die erste leitfähige Schicht 2 als Titanschicht ausgebildet.

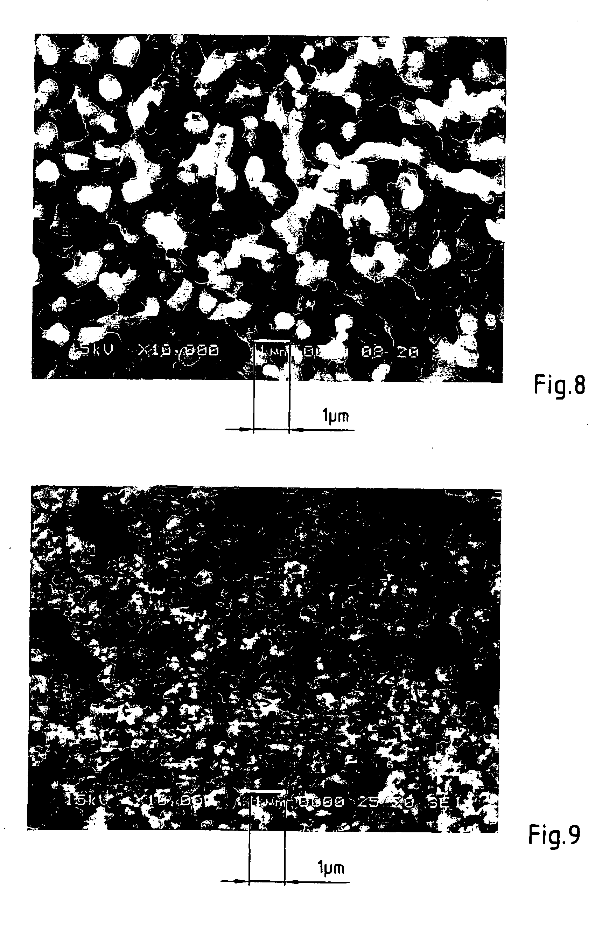

Die auf der ersten leitfähigen Schicht 2 aufgebrachte oder erzeugte Metalloxidschicht 3 hat eine schwammartig poröse Struktur mit einem Porendurchmesser von 50 nm bis 1000 nm, vorliegend ca. 300 nm, und weist eine Dicke von 100 nm bis 5 µm, vorliegend ca. 1000 nm auf. Vorliegend ist die Metalloxidschicht 3 als Titanoxidschicht TiOₓ, speziell als Titandioxidschicht in einer Rutil-Kristallform, ausgebildet und durch Anodisierung der Oberfläche der darunter liegenden Titanschicht 2 bei einer Anodisierspannung von 120 V in 3,5 molarer Schwefelsäure erzeugt. Eine elektronenmikroskopische Aufnahme der anodisierten Titanschicht 2 ist in Fig. 8 gezeigt. Zusätzlich kann auf die durch Anodisierung der ersten leitfähigen Schicht 2 erzeugte schwammartig poröse Metalloxidschicht 3 eine weitere schwammartig poröse Metalloxidschicht durch einen Sol-Gel-Prozess aufgebracht werden (hier nicht dargestellt).

Auf die schwammartig poröse Titandioxidschicht 3 ist, wie oben erwähnt, eine Metallschicht 4 als Frontelektrode der Dioden-Dünnschichtanordnung 1 aufgebracht. Dies kann in einem Vakuumprozess (PVD-Verfahren), beispielsweise durch Sputtern, oder durch eine galvanische Abscheidung erfolgt sein. Insbesondere handelt es sich bei der Metallschicht 4 um eine Platin-, Palladium- oder Goldschicht. Insbesondere diese Elemente weisen eine höhere Elektronenaustrittsarbeit als die Titandioxidschicht 3 auf. Vorliegend ist die Metallschicht 4 als Platinschicht ausgebildet, welche infolge der Art des Aufbringens auf die schwammartig poröse Metalloxidschicht 3 ebenfalls eine poröse Struktur aufweist. Die Dicke der Metallschicht 4 beträgt typischerweise 5 bis 100 nm, vorliegend ca. 50 nm. Fig. 9 zeigt eine elektronenmikroskopische Aufnahme der anodisierten Titanschicht 2 mit galvanisch aufgebrachter poröser Platinschicht 4. Eine elektronenmikroskopische Untersuchung porösen Platinschicht 4 zeigt, dass diese aus Kristalliten mit einem Durchmesser zwischen 5 und 100 nm besteht. Aufgrund eines verbesserten Signal-Rausch-Verhältnisses der Rasterelektronenmikroskopaufnahme ist mit aufgesputtertem Platin eine feiner aufgelöste Struktur zu erkennen.

Die Metallschicht 4 kann zusätzlich noch mit einer Schutzschicht (nicht dargestellt) versehen sein, bevorzugt mit einer Tetraethylorthosilikat-, Hexamethyldisiloxan-oder Hexamethyldisilazan-schicht.

In Fig. 2 ist ein Wasserstoffsensor 10 mit der Dioden-Dünnschichtanordnung 1 aus Fig. 1 in perspektivischer Darstellung gezeigt. Die Rückelektrode wird hierbei selbsttragend durch eine Titanfolie 20 mit einer Dicke von typischerweise 0,25 mm gebildet. Eine typische Flächenabmessung für die Rückelektrode ist 3,5 x 7 mm². Durch einseitige und vollflächige Anodisierung der Titanfolie 20 ist auf der Oberfläche der Titanfolie 20 wiederum eine schwammartige und nano-offenporige Titandioxidschicht 30 in Rutil-Kristallform erzeugt, auf der eine Platinschicht 40 aufgesputtert oder galvanisch abgeschieden ist. Durch entsprechende Maskierung der Titandioxidschicht 30 können die Abmessungen der Platinschicht 40, d.h. die Abmessungen der Sensorfläche 41, festgelegt werden. Typische Abmessungen sind hier 5 x 5 mm². Hierdurch wird ein sehr robuster Sensor 10 zur Bestimmung des Wasserstoffgehalts in umgebender Luft oder in einem umgebenden Gas bereitgestellt. Die Platinschicht 40 ist vorliegend durch konventionelle Kontaktierungsmethoden mit einer Frontelektroden-Kontaktfläche 42 kontaktiert. Diese kann beispielsweise durch Silberleitpaste gebildet werden. Gleiches gilt für die Kontaktierung der Rückelektrode. Elektrische Anschlüsse 23, 43 können mit den Kontaktflächen der Elektroden (Titanschicht 20 und Platinschicht 40) durch Löten, Ultraschallschweißen, Drahtbonden oder Reibschweißen hergestellt werden, wodurch langzeitstabile ohmsche Kontakte realisiert werden. Über die elektrischen Anschlüsse 23, 43 ist der Wasserstoffsensor mit einer - nicht dargestellten - Ansteuerungs- und Auswerteeinheit verbunden, der einerseits die Funktion der Energieversorgung der Dioden-Dünnschichtanordnung und andererseits die Funktion einer schnellen Auswerteelektronik zukommt.

Im Betrieb des Wasserstoffsensors 10 trifft das Gas G, dessen Wasserstoffgehalt zu bestimmen ist, oberflächig auf die Sensorfläche 41 auf, umspült diese oder wird an der Sensorfläche 41 vorbeigeführt. Wie Untersuchungen des Erfinders gezeigt haben, lassen sich mit einem solchen Sensor Wasserstoffkonzentrationen in einem Bereich von typischerweise 0,1 bis 4,4 Vol.-% präzise messen.

Stets zu berücksichtigen ist die Umgebungstemperatur und die Luftfeuchtigkeit, da diese die Wasserstoffkonzentrationsmessung beeinflussen. Mit dem vorstehend beschriebenen Wasserstoffsensor 10 können jedoch die drei Sensoren durch ein einziges Element realisiert werden. So kann vor der Messung an die Titanschicht 2 (Rückelektrode) oder an die Platinschicht 4 (Frontelektrode) lateral eine Spannung (bevorzugt 12 V) angelegt und der Strom, der durch die hochohmige Titanschicht 2 fließt, gemessen werden. Der Stromfluss ist temperaturabhängig, so dass nach entsprechender Kalibrierung von dem fließenden Strom auf die Temperatur geschlossen werden kann. Durch den Stromfluss wird die Dioden-Dünnschichtanordnung 1 kurzfristig aufgeheizt, so dass adsorbiertes Wasser, was sich aus der Luftfeuchtigkeit auf dem Wasserstoffsensor 10 niedergeschlagen hat, desorbiert wird und dementsprechend die Messung der Wasserstoffkonzentration nicht mehr beeinflusst. Es versteht sich, dass das vorstehende Messprinzip auch bei Dioden-Dünnschichtanordnungen zur Anwendung kommen kann, die eine andere Schichtzusammensetzung aufweisen.

Die Dioden-Dünnschichtanordnung 1 und entsprechend der Wasserstoffsensor 10 der Fig. 2 können zudem als elektrische Energiequelle verwendet werden, da sie ohne externe Stromversorgung ab einem gewissen Schwellwert von Wasserstoff und im Beisein von Restsauerstoff ein elektrisches Signal liefern, das in einer nachgeschalteten Elektronik für Steuerungs- und Alarmfunktionen verwendet werden kann. Hierbei liegt der Schwellwert von Wasserstoff sinnvollerweise unterhalb der Explosionsgefahr des Gasgemisches.

Die Ausführungsform des Wasserstoffsensors 100 gemäß Fig. 3 unterscheidet sich von der der Fig. 2 dadurch, dass die Titanschicht 200 erfindungsgemäß nicht selbsttragend ausgeführt, sondern auf ein Glas-, Silizium- oder Keramiksubstrat 500 aufgebracht ist, beispielsweise durch Sputtern, Aufdampfen, galvanische Abscheidung oder in einem Sol-Gel-Prozess. Neben Titan können auch Fluor-dotiertes Zinnoxid oder auch Tantal, Wolfram, Kupfer, Zinn oder ein anderes Übergangs- oder Hauptgruppen-Metall als Material für die die Rückelektrode bildende erste leitfähige Schicht verwendet werden. Stets muss dabei das verwendete Material eine kleinere Elektronenaustrittsarbeit aufweisen als die auf die Schicht aufgebrachte schwammartig poröse Metalloxidschicht, vorliegend Titandioxid, so dass sich ein ohmscher Kontakt bildet, der die bei der chemischen Reaktion an den wirksamen Oberflächen der Dioden-Dünnschichtanordnung entstehenden elektrischen Ladungen sammelt.

Die Titandioxidschicht 300 als Metalloxidschicht ist in dem Ausführungsbeispiel der Fig. 3 durch Anodisierung der Titanschicht 200 ggf. ergänzt durch ein Sol-Gel-Verfahren aufgebracht. Darauf ist in der vorstehend im Zusammenhang mit Fig. 2 beschriebenen Weise eine Platinschicht 400 aufgebracht.

Vorliegend sind die einzelnen Schichten 200, 300, 400 der Dioden-Dünnschichtanordnung des Wasserstoffsensors 100 überlappend ausgebildet, so dass, wie dargestellt, an den Randseiten der Frontelektrode (Platinschicht 400) und der Rückelektrode (Titanschicht 200) Kontaktierstreifen 220, 420 angeordnet werden können. Die Kontaktierstreifen 220, 420 können wiederum aus Leitsilber gebildet werden, wobei die Anschlüsse 230, 430 mittels Löten, Ultraschall, Reibschweißen oder mittels Drahtbonden realisiert werden können. Ferner kann ein elektrisch leitender polymerer Kleber für die Herstellung der Anschlüsse 230, 430 verwendet werden. Durch die Ausbildung der beiden Kontaktierstreifen 220, 420 in Form von Leitsilber kann ein SMD (Surface-Mounted-Device) Baustein hergestellt werden. Bei Verwendung einer aktiven Sensorfläche 210 von beispielsweise 5 x 5 mm² kann eine SMD Gehäusegröße mit dem metrischen Code 5750, also 5,7 mm Länge und 5,0 mm Breite eingesetzt werden.

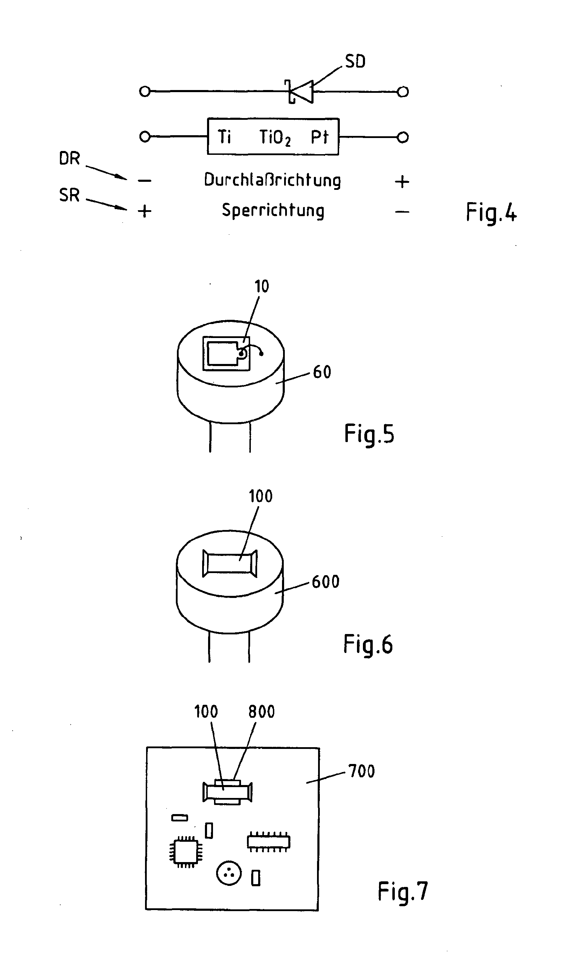

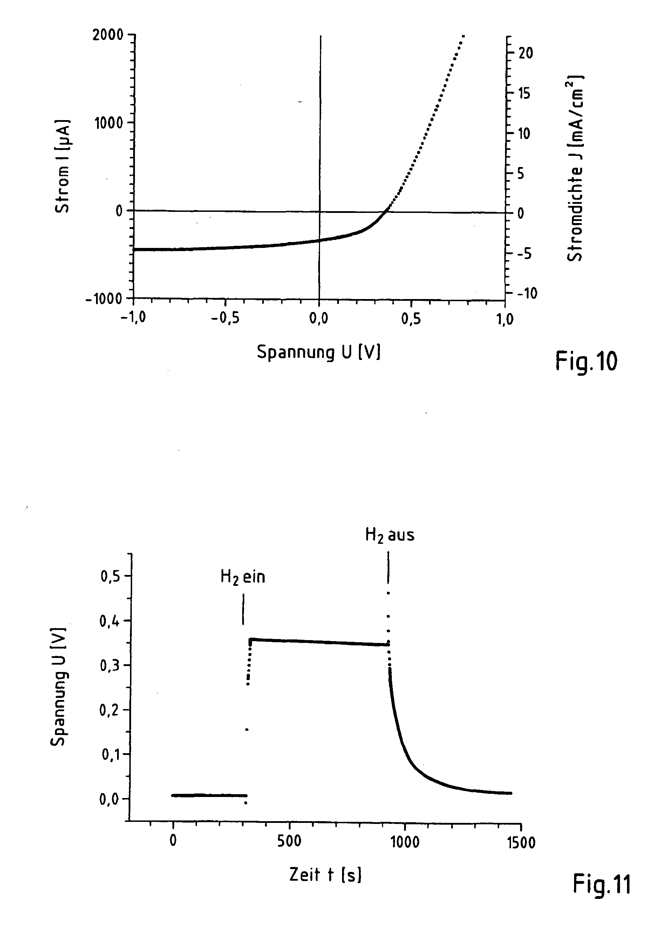

Fig. 4 zeigt ein Ersatzschaltbild für einen Wasserstoffsensor, wobei die Dioden-Dünnschichtanordnung 1 mit einem gewöhnlichen Schottky-Dioden-Schaltsymbol SD dargestellt ist. Der bevorzugte Schichtaufbau der Dioden-Dünnschichtanordnung 1 (Titanschicht/Titandioxidschicht/Platinschicht) ist ebenfalls dargestellt. Er zeichnet sich bei entsprechender Polung durch eine nichtlineare Strom-Spannungskennlinie (vgl. Fig. 10) mit einer starken Sensitivität für Wasserstoff aus. Die Diode wird in Sperrrichtung SR betrieben, wenn die Platinschicht als Frontelektrode negativ gepolt ist. Ein Betrieb des Wasserstoffsensors in Sperrrichtung SR ermöglicht keinen Betrieb bei tiefen Temperaturen und weist hier eine weitgehend lineare Kennlinie mit vergleichsweise geringer Sensitivität auf. Bei dem Betrieb der Dioden-Dünnschichtanordnung 1 in Durchlassrichtung DR mit positiv gepolter Frontelektrode ist hingegen die Strom-Spannungskennlinie stark nichtlinear und eine Sensorfunktion ist mindestens in einem Bereich von -40°C bis über +125°C gegeben.

Fig. 5 zeigt einen in ein TO-Gehäuse 60 integrierten Wasserstoffsensor 10 in schematisierter perspektivischer Darstellung. Dabei kann der Wasserstoffsensor einzeln, in Form eines Arrays oder auch gemeinsam mit einer elektronischen Ansteuerungs- und Auswerteeinheit (nicht dargestellt) in dem TO-Gehäuse 60 angeordnet sein. Das TO-Gehäuse 60 kann mit einer wasserundurchlässigen und dampfdiffusionsoffenen beziehungsweise gasdurchlässigen Membran, beispielsweise Gore-Tex (W. L. Gore & Associates) abgedeckt sein (nicht dargestellt). Ferner kann im Innenbereich des TO-Gehäuses 60 ein Molekularsieb für Wasserdampf in Form von beispielsweise Zeolit-Kügelchen mit 3 Angström Porendurchmesser eingebaut sein (nicht dargestellt), so dass auf diese Weise Wasserdampf vom Wasserstoffsensor 10 ferngehalten wird. Die Zeolit-Kügelchen können ausheizbar ausgeführt sein. Alternativ der ergänzend zum Einsatz von Zeolit-Kügelchen können auch geeignete Gettersysteme mit der Eigenschaft der Adsoption von Wasserdampf verwendet werden.

Fig. 6 zeigt einen in ein TO-Gehäuse 600 integrierten Wasserstoffsensor 100 in Form eines SMD-Bauteils in schematisierter perspektivischer Darstellung. Die in Zusammenhang mit Fig. 5 beschriebenen speziellen Ausgestaltungen in Bezug auf Verhinderung der Wasserdampfbeaufschlagung des Wasserstoffsensors 100 sind auch hier realisierbar. Die in das TO-Gehäuse 600 integrierbare Ansteuerungs- und Auswerteelektronik ist aus Gründen der Übersichtlichkeit nicht im Einzelnen dargestellt. Insbesondere können eine Stromversorgung in Form einer Batterie oder eines Akkumulators vorgesehen sein, welche einen Stromausfall kompensieren können bzw. ermöglichen, dass der Wasserstoffsensor 100 weitgehend ohne externe Stromversorgung auskommt.

Fig. 7 zeigt den Wasserstoffsensor 100 der Figur 2 als SMD-Bauteil auf einer Platine 700. Vorliegend ist in die Platine 700 eine Ausnehmung 800 eingeformt, über der, wie dargestellt, der Wasserstoffsensor 100 angeordnet ist. Im Betrieb des Wasserstoffsensors 100 kann durch die Ausnehmung 800 in der Platine 700 ein Gas strömen, dessen Wasserstoffgehalt sodann durch den Wasserstoffsensor 100 bestimmt wird. Vorliegend ist der Wasserstoffsensor 100 liegend über der Ausnehmung 800 angeordnet. Ebenso kann er jedoch auch hochkant angeordnet werden, um den Strömungswiderstand zu reduzieren. Die Platine 700 kann als übliche Leiterplatte auf Basis von FR-2, FR-3, FR-4 oder CEM-1, CEM-2 oder CEM-3 Material oder dergleichen als einseitige oder doppelseitige Platine, Multilayerplatine oder Starr-Flex oder Flex-Platine ausgebildet sein, wobei neben dem Wasserstoffsensor auch andere, evtl. mit dem Wasserstoffsensor 100 zusammenwirkende elektronischen Bauelemente oder Sensoren angeordnet sein können. Die Platine 700 kann ferner aus einem Keramiksubstrat in nach Art einer Dickfilmschaltung hergestellt sein. Für die korrekte Kalibration und Funktionsweise des Wasserstoffsensors 100 kann es erforderlich sein, eine Temperaturkompensation und eine Wasserdampfkompensation durchzuführen. Hierzu kann ein Temperatursensor oder ein Wasserdampfsensor auf der Platine 700 in der Nähe des Wasserstoffsensors 100 angeordnet sein, so dass die durch den Wasserstoffsensor 100 gemessene Wasserstoffkonzentration bezüglich der jeweils gegebenen Temperatur und Wasserdampfkonzentration korrigiert werden kann. Es versteht sich, dass im Falle der Konstanthaltung der Temperatur und der Wasserdampfkonzentration im Bereich des Wasserstoffsensors 100 auf derartige zusätzliche Sensoren verzichtet werden kann. Übliche Trocknungsmittel in Form des bereits erwähnten Molekularsiebs mit typischerweise 3 Angström Porengröße, beispielsweise in Form von Zeolit-Kügelchen, können ohne weiteres auf oder im Bereich der Platine 700 und damit in der Nähe des Wasserstoffsensors 100 vorgesehen sein (nicht dargestellt). Ferner kann das Molekularsieb (nicht dargestellt) ausheizbar ausgebildet sein.

Fig. 10 zeigt eine der Dioden-Dünnschichtanordnung 1 aus Fig. 1 bei der Umspülung der Dioden-Dünnschichtanordnung 1 mit den Reaktanden Wasserstoff und Sauerstoff. Im Einzelnen ist die mit einem Potentiostaten bestimmte Strom-Spannungskennlinie dargestellt, wobei der Strom I (in µA) und die Stromdichte J als Funktion der an die Platinschicht 4 angelegten Spannung U aufgetragen ist. Die gemessene Dioden-Dünnschichtanordnung 1 weist hierbei die im Zusammenhang mit Fig. 1 beschriebene Schichtstruktur Ti/TiO_{2/}Pt auf. Der die Dioden-Dünnschichtanordnung 1 umspülende Gasstrom beinhaltet 3,5 % H₂ und 6% O₂ in Stickstoff bei Raumtemperatur. Als Rampengeschwindigkeit des Potentiostaten ist dU/dt = 0,1 V/s gewählt. Negative Werte des Stromes I entsprechen einem Strom aus negativer Ladung in einem äußeren Stromkreis um die Dioden-Dünnschichtanordnung 1 von der Titanschicht 2 zur Platinschicht 4. Die Stromdichte J ergibt sich als J = I/A aus dem Strom I und der geometrischen Fläche A des Platin-Kontaktes.

Wie in Fig. 10 erkennbar, schneidet die Strom-Spannungskennlinie die Ordinate (Stromachse) bei I₀ < 0 und die Abszisse (Spannungsachse) bei U₀ > 0. Hierbei bedeuten negative Werte des Stromes, dass negative Ladungen im äußeren Stromkreis von der Titanschicht 2 zur Platinschicht 4 fließen, und positive Werte der Spannung, dass die Platinschicht 4 positiv gepolt ist. Diese Kenngrößen zeigen, dass die Dioden-Dünnschichtanordnung 1 bei Ablauf einer chemischen Reaktion zwischen den Reaktanden Wasserstoff und Sauerstoff eine elektromotorische Kraft entwickelt und damit die Dioden-Dünnschichtanordnung 1 als elektrische Energiequelle verwendet werden kann.

Fig. 11 zeigt eine Spannungstransiente über die Zeit aufgetragen bei der Umspülung der Dioden-Dünnschichtanordnung 1 aus Fig. 1 in einem Wasserstoff und Sauerstoff enthaltenen Gasstrom. In dem Moment, in dem die Reaktanden an der Oberfläche der Platinschicht 4 miteinander eine chemische Reaktion eingehen, erhöht sich die Spannung schlagartig. Im Einzelnen wurde der Transient der Spannung U₀ zwischen der Titanschicht 2 und der Platinschicht 4 (nahezu stromlos bestimmt mit hochohmigen Digital-Voltmeter) bei Umspülung der Dioden-Dünnschichtanordnung 1 mit 3,5% H₂ und 6% O₂ in Stickstoff gemessen. Dabei markiert der Beginn der Umspülung "H₂ ein" und das Ende "H₂ aus".

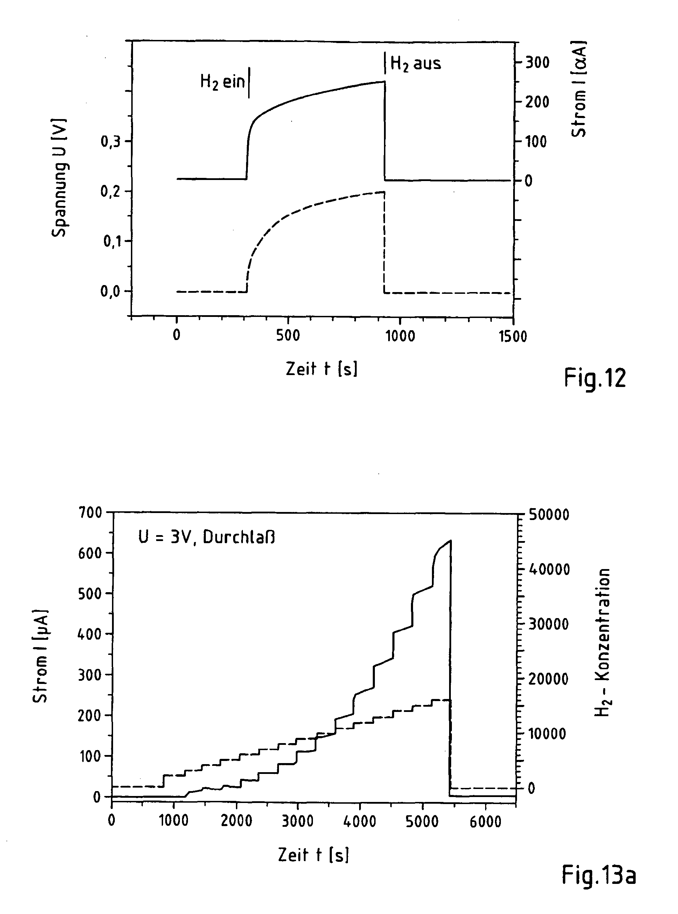

Fig. 12 zeigt die Strom- und Spannungstransienten der Dioden-Dünnschichtanordnung 1 aus Fig. 1 in einem Wasserstoff und Sauerstoff enthaltenen Gasstrom. Hierbei wurden der Strom mit einem Digital-Amperemeter mit endlichem Innenwiderstand und die Spannung an der Dioden-Dünnschichtanordnung 1 stromlos mit einem hochohmigen Digital-Voltmeter bestimmt. Der Strom I bei Umspülung mit den Reaktanden H₂, O₂ ist kleiner als der potentiostatisch bei U=0 bestimmte Strom I₀, da sich bei dieser Art von Messung während der Reaktion auch die Spannung ändert. Der Transient des Produktes aus Strom und Spannung zeigt, dass während der Reaktion an der Dioden-Dünnschichtanordnung 1 eine elektrische Leistung entsteht. Gleichzeitig entsteht an der Dioden-Dünnschichtanordnung 1 infolge der Umsetzung des Wasserstoffs und Sauerstoffs zu Wasser Reaktionswärme. Die resultierende Temperaturerhöhung an der Dioden-Dünnschichtanordnung 1 führt dazu, dass Strom-und Spannungswerte mit der Zeit ansteigen.

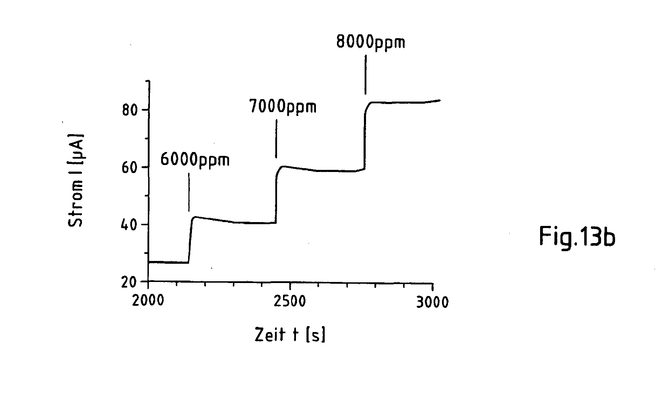

Die Fig. 13a und 13b zeigen schließlich den Verlauf des Stroms in Abhängigkeit von der Zeit bei einer Umspülung der Dioden-Dünnschichtanordnung 1 aus Fig. 1 mit Luft, in der Wasserstoff unterschiedlicher Konzentration enthalten ist. Im Einzelnen werden unterschiedliche Wasserstoffkonzentrationen im Bereich 2000 bis 16000 ppm (in Stufen von 1000 ppm) eingestellt. Die Dioden-Dünnschichtanordnung 1 kann für diese Anwendung auch an eine äußere Spannungsquelle angeschlossen werden. Wie erkennbar, hängt der Strom eindeutig von der Wasserstoffkonzentration ab; die Detaildarstellung des Konzentrationsbereichs 5000 bis 8000 ppm der Fig. 13b zeigt, dass die Dioden-Dünnschichtanordnung 1 mit kurzen Ansprechzeiten auf eine Veränderung der H₂-Konzentration reagiert.

## Patentansprüche

1. Wasserstoffsensor mit einer Dioden-Dünnschichtanordnung (1) zur Detektion von Wasserstoff, umfassend:
- eine erste leitfähige Schicht (2),
- eine auf die erste leitfähige Schicht (2) aufgebrachte oder auf der ersten leitfähigen Schicht (2) erzeugte Metalloxidschicht (3), und
- eine auf die Metalloxidschicht (3) aufgebrachte Metallschicht (4),
- wobei die Elektronenaustrittsarbeit der ersten leitfähigen (2) Schicht niedriger ist als die Elektronenaustrittsarbeit der Metalloxidschicht (3), derart, dass zwischen der ersten leitfähigen Schicht (2) und der Metalloxidschicht (3) ein ohmscher Kontakt gebildet ist, und
- wobei zwischen der Metalloxidschicht (3) und der Metallschicht (4) ein Schottky-Kontakt gebildet ist, und
- wobei die Metalloxidschicht (3) eine schwammartig poröse Struktur aufweist und die auf die Metalloxidschicht (3) aufgebrachte Metallschicht (4) eine entsprechende poröse Struktur aufweist,
**dadurch gekennzeichnet, dass**
- die erste leitfähige Schicht (200) als nicht selbsttragende Schicht auf einem Substrat, insbesondere einem Glassubstrat oder einem Siliziumsubstrat oder einem Keramiksubstrat, ausgebildet ist und
- die erste leitfähige Schicht (2, 200) und die poröse Metallschicht (4, 400) mit einer elektronischen Ansteuerungs- und Auswerteeinheit verbunden sind, die dazu ausgebildet ist, zur Bestimmung der Temperatur der ersten leitfähigen Schicht (20, 200) lateral an die erste leitfähige Schicht (20, 200) eine Spannung anzulegen.

2. Wasserstoffsensor (10, 100) nach Anspruch 1, **dadurch gekennzeichnet, dass**
- die erste leitfähige Schicht (2) eine Schicht aus Kupfer, Tantal, Titan, Wolfram, Zinn oder einer Legierung hieraus oder eine TCO-Schicht, insbesondere eine Schicht aus Fluor-dotiertem Zinnoxid, ist
und/oder
- die schwammartig poröse Metalloxidschicht (3) einen Porendurchmesser von 50 nm bis 1000 nm, bevorzugt ca. 300 nm, aufweist und/oder die Dicke der schwammartig porösen Metalloxidschicht (3) 100 nm bis 5 µm, bevorzugt 1 µm, beträgt.

3. Wasserstoffsensor (10, 100) nach einem der Ansprüche 1 oder 2, **dadurch gekennzeichnet, dass**
- die schwammartig poröse Metalloxidschicht (3) eine Titanoxid-Schicht, insbesondere eine TiO₂-Schicht, ist
und/oder
- die erste leitfähige Schicht (2) eine Titanschicht ist und die schwammartig poröse Metalloxidschicht (3) eine durch Anodisieren der Oberfläche der ersten leitfähigen Schicht (2) gebildete Titanoxidschicht ist
und/oder
- eine schwammartig poröse Metalloxidschicht mittels Sol-Gel-Prozess auf der durch Anodisierung der ersten leitfähigen Schicht erzeugten schwammartig porösen Metalloxidschicht (3) abgeschieden ist
und/oder
- die poröse Metallschicht (4) eine Platin-, Palladium- oder Goldschicht ist und/oder
- die poröse Metallschicht (4) eine kristallitische Struktur aufweist, wobei die Kristallite einen Durchmesser von 5 nm bis 100 nm aufweisen.

4. Wasserstoffsensor (10, 100) nach einem der Ansprüche 1 bis 3, **dadurch gekennzeichnet, dass**
- die erste leitfähige Schicht (20, 200) mit dem Minuspol und die poröse Metallschicht (40, 400) mit dem Pluspol der elektronischen Ansteuerungs- und Auswerteeinheit verbunden ist, so dass die Dioden-Dünnschichtanordnung in Durchlassrichtung betrieben wird,
oder
- die erste leitfähige Schicht (20, 200) mit dem Pluspol und die poröse Metallschicht (40, 400) mit dem Minuspol der elektronischen Ansteuerungs- und Auswerteeinheit verbunden ist, so dass die Dioden-Dünnschichtanordnung in Sperrrichtung betrieben wird.

5. Wasserstoffsensor (10, 100) nach einem der Ansprüche 1 bis 4, **dadurch gekennzeichnet, dass**
- der Wasserstoffsensor (10, 100) in ein TO-Gehäuse (60, 600) eingebaut oder Teil eines Leadframe- oder LCC-Packages ist, wobei das TO-Gehäuse (60, 600) insbesondere ein Molekularsieb für Wasserdampf oder eine wasserundurchlässige und dampfdiffusionsoffene und/oder gasdurchlässige Membran, insbesondere eine PTFE-Membran oder eine "Pall-Membran", umfasst,
oder
- der Wasserstoffsensor ein SMD-Bauteil ist.

6. Verfahren zur Herstellung eines Wasserstoffsensors mit einer Dioden-Dünnschichtanordnung (1) zur Detektion von Wasserstoff nach einem der Ansprüche 1 bis 5, umfassend folgende Verfahrensschritte:
- Bereitstellen der ersten leitfähigen Schicht (2),
- Aufbringen der Metalloxidschicht (3) auf die erste leitfähige Schicht (2) oder Erzeugen der Metalloxidschicht (3) auf der ersten leitfähigen Schicht (2), wobei die Metalloxidschicht (3) derart durch ein elektrochemisches Verfahren oder durch einen Sol-Gel-Prozess aufgebracht oder erzeugt wird, dass sie eine schwammartig poröse Struktur ausbildet,
- Aufbringen der Metallschicht (4) auf die Metalloxidschicht (3), wobei die Metallschicht (4) derart durch ein elektrochemisches Verfahren oder durch ein Vakuumabscheideverfahren auf die Metalloxidschicht (3) aufgebracht wird, dass die auf die Metalloxidschicht (3) aufgebrachte Metallschicht (4) eine der schwammartig porösen Struktur der Metalloxidschicht (3) entsprechende poröse Struktur aufweist,
**dadurch gekennzeichnet, dass**
- die erste leitfähige Schicht (2) als nicht selbsttragende Schicht auf einem Substrat, insbesondere einem Glassubstrat oder einem Siliziumsubstrat oder einem Keramiksubstrat, ausgebildet wird und
- die erste leitfähige Schicht (2, 200) und die poröse Metallschicht (4, 400) mit einer elektronischen Ansteuerungs- und Auswerteeinheit verbunden werden, die dazu ausgebildet ist, zur Bestimmung der Temperatur der ersten leitfähigen Schicht (20, 200) lateral an die erste leitfähige Schicht (20, 200) eine Spannung anzulegen.

7. Verfahren nach Anspruch 6, **dadurch gekennzeichnet, dass**
- die erste leitfähige Schicht (2) eine Titanschicht ist und die Metalloxidschicht (3) durch Anodisierung auf der Titanschicht (2) erzeugt wird, wobei die Anodisierung der Titanschicht (2) bei einer Anodisierspannung von 90 bis 200 V, bevorzugt ca. 120 V, erfolgt, wobei die Anodisierung insbesondere in 0,5 bis 14 molarer Schwefelsäure, bevorzugt ca. 3,5 molarer Schwefelsäure, erfolgt, oder
- die Metalloxidschicht (3) mittels PVD auf die ersten leitfähigen Schicht (2) aufgebracht wird.

8. Verfahren nach Anspruch 7, **dadurch gekennzeichnet, dass**
- auf die durch Anodisierung der ersten leitfähigen Schicht (2) erzeugte schwammartig poröse Metalloxidschicht (3) eine weitere schwammartig poröse Metalloxidschicht durch einen Sol-Gel-Prozess aufgebracht wird, wobei
- die schwammartig poröse Metalloxidschicht (3) und die weitere schwammartig poröse Metalloxidschicht insbesondere chemisch identisch sind.

9. Verfahren nach einem der Ansprüche 6 bis 8, **dadurch gekennzeichnet, dass** die Metallschicht (4) mittels eines PVD-Verfahrens, insbesondere mittels Sputtern oder Aufdampfen, auf die schwammartig poröse Metalloxidschicht (3) aufgebracht wird.

10. Verfahren nach einem der Ansprüche 6 bis 9, **dadurch gekennzeichnet, dass** die Metallschicht (4) mit einer Schutzschicht, insbesondere mit einer Tetraethylorthosilikat-, Hexamethyldisiloxan- oder Hexamethyldisilazanschicht, versehen wird.

11. Verwendung eines Wasserstoffsensors (10, 100) gemäß einem der Ansprüche 1 bis 5 als Wasserstoffsensor in einem mit Wasserstoff betriebenen Fahrzeug oder in Wasserstofftanks und -leitungen zur Erfassung von Leckagen.

12. Verwendung eines Wasserstoffsensors (10, 100) gemäß einem der Ansprüche 1 bis 5 als Wasserstoffsensor in einer Sensoranordnung zur Messung der Zusammensetzung von Gasen, insbesondere von Abgasen oder Stadtgas.

13. Verwendung eines Wasserstoffsensors (10, 100) gemäß einem der Ansprüche 1 bis 5 in einer Feuermeldereinheit.

14. Verwendung eines Wasserstoffsensors (10, 100) gemäß einem der Ansprüche 1 bis 5 als elektrische Energiequelle, speziell als Dünnschicht-Einkammer-Festoxid-Brennstoffzelle.

15. Verwendung eines Wasserstoffsensors (10, 100) gemäß einem der Ansprüche 1 bis 5 als UV-Sensor, der für sichtbares Licht im Wesentlichen unempfindlich ist.

## Claims

1. Hydrogen sensor with a diode thin-film arrangement (1) for detecting hydrogen, comprising:
- a first conductive layer (2),
- a metal oxide layer (3) applied onto the first conductive layer (2) or produced on the first conductive layer (2),
- a metal layer (4) applied onto the metal oxide layer (3),
- wherein the electron work function of the first conductive layer (2) is lower than the electron work function of the metal oxide layer (3) such that an ohmic contact is formed between the first conductive layer (2) and the metal oxide layer (3), and
- wherein a Schottky contact is formed between the metal oxide layer (3) and the metal layer (4), and
- wherein the metal oxide layer (3) has a sponge-like, porous structure and the metal layer (4) applied onto the metal oxide layer (3) has a corresponding porous structure,
**characterised in that**
- the first conductive layer (200) is formed as a non self-supporting layer on a substrate, in particular a glass substrate or a silicon substrate or a ceramic substrate, and
- the first conductive layer (2, 200) and the porous metal layer (4, 400) are connected to an electronic control and evaluation unit which is configured to apply a voltage laterally to the first conductive layer (20, 200) in order to determine the temperature of the first conductive layer (20, 200).

2. Hydrogen sensor (10, 100) according to Claim 1, **characterised in that**
- the first conductive layer (2) is a layer made of copper, tantalum, titanium, tungsten, tin or an alloy thereof or a TCO layer, in particular a layer made of fluorine-doped tin oxide,
and/or
- the sponge-like, porous metal oxide layer (3) has a pore diameter of 50 nm to 1000 nm, preferably about 300 nm, and/or the thickness of the sponge-like, porous metal oxide layer (3) is 100 nm to 5 µm, preferably 1 µm.

3. Hydrogen sensor (10, 100) according to either of Claims 1 and 2, **characterised in that**
- the sponge-like, porous metal oxide layer (3) is a titanium oxide layer, in particular a TiO₂ layer,
and/or
- the first conductive layer (2) is a titanium layer and the sponge-like, porous metal oxide layer (3) is a titanium oxide layer formed by anodising the surface of the first conductive layer (2)
and/or
- a sponge-like, porous metal oxide layer is deposited by means of a sol-gel process on the sponge-like, porous metal oxide layer (3) produced by anodising the first conductive layer
and/or
- the porous metal layer (4) is a platinum, palladium or gold layer
and/or
- the porous metal layer (4) has a crystallite structure, wherein the crystallizes have a diameter of 5 nm to 100 nm.

4. Hydrogen sensor (10, 100) according to any one of Claims 1 to 3, **characterised in that**
- the first conductive layer (20, 200) is connected to the negative terminal of the electronic control and evaluation unit and the porous metal layer (40, 400) is connected to the positive terminal of the electronic control and evaluation unit, so that the diode thin-film arrangement is operated in the forward direction,
or
- the first conductive layer (20, 200) is connected to the positive terminal of the electronic control and evaluation unit and the porous metal layer (40, 400) is connected to the negative terminal of the electronic control and evaluation unit, so that the diode thin-film arrangement is operated in the reverse direction.

5. Hydrogen sensor (10, 100) according to any one of Claims 1 to 4, **characterised in that**
- the hydrogen sensor (10, 100) is fitted into a TO housing (60, 600) or is part of a lead frame or an LCC package, wherein the TO housing (60, 600) in particular comprises a molecular sieve for water vapour or a membrane impermeable to water and open to vapour diffusion and/or permeable to gas, in particular a PTFE membrane or a "pall membrane",
or
- the hydrogen sensor is an SMD component.

6. Method for producing a hydrogen sensor with a diode thin-film arrangement (1) for defecting hydrogen according to any one of Claims 1 to 5, comprising the following method steps:
- providing the first conductive layer (2),
- applying the metal oxide layer (3) onto the first conductive layer (2) or producing the metal oxide layer (3) on the first conductive layer (2), wherein the metal oxide layer (3) is applied or produced by an electrochemical process or by a sol-gel process in such a way that it forms a sponge-like, porous structure,
- applying the metal layer (4) onto the metal oxide layer (3), wherein the metal layer (4) is applied onto the metal oxide layer (3) by an electrochemical process or by a vacuum deposition process in such a way that the metal layer (4) applied onto the metal oxide layer (3) has a porous structure corresponding to the sponge-like, porous structure of the metal oxide layer (3),
**characterised in that**
- the first conductive layer (2) is formed as a non self-supporting layer on a substrate, in particular a glass substrate or a silicon substrate or a ceramic substrate, and
- the first conductive layer (2, 200) and the porous metal layer (4, 400) are connected to an electronic control and evaluation unit which is configured to apply a voltage laterally to the first conductive layer (20, 200) in order to determine the temperature of the first conductive layer (20, 200).

7. Method according to Claim 6, **characterised in that**
- the first conductive layer (2) is a titanium layer and the metal oxide layer (3) is produced by anodising the titanium layer (2), wherein the titanium layer (2) is anodised at an anodising voltage of 90 to 200 V, preferably about 120 V, wherein anodising in particular takes place in 0.5 to 14 molar sulphuric acid, preferably in about 3.5 molar sulphuric acid,
or
- the metal oxide layer (3) is applied onto the first conductive layer (2) by means of PVD.

8. Method according to Claim 7, **characterised in that**
- a further sponge-like, porous metal oxide layer is applied by means of a sol-gel process onto the sponge-like, porous metal oxide layer (3) produced by anodising the first conductive layer (2), wherein
- the sponge-like, porous metal oxide layer (3) and the further sponge-like, porous metal oxide layer in particular are chemically identical.

9. Method according to any one of Claims 6 to 8, **characterised in that** the metal layer (4) is applied onto the sponge-like, porous metal oxide layer (3) by means of a PVD process, in particular by sputtering or vapour deposition.

10. Method according to any one of Claims 6 to 9, **characterised in that** the metal layer (4) is provided with a protective layer, in particular with a tetraethylorthosilicate, hexamethyldisiloxane or hexamethyldisilazane layer.

11. Use of a hydrogen sensor (10, 100) according to any one of Claims 1 to 5 as a hydrogen sensor for detecting leaks in a vehicle running on hydrogen or in hydrogen tanks and pipes.

12. Use of a hydrogen sensor (10, 100) according to any one of Claims 1 to 5 as a hydrogen sensor in a sensor arrangement for measuring the composition of gases, in particular exhaust gases or town gas.

13. Use of a hydrogen sensor (10, 100) according to any one of Claims 1 to 5 in a fire alarm unit.

14. Use of a hydrogen sensor (10, 100) according to any one of Claims 1 to 5 as an electric energy source, particularly as a thin-film single chamber solid oxide fuel cell.

15. Use of a hydrogen sensor (10, 100) according to any one of Claims 1 to 5 as a UV sensor which is essentially insensitive to visible light.

## Revendications

1. Détecteur d'hydrogène avec un ensemble à couches minces à diode (1) pour la détection d'hydrogène, comportant:
- une première couche conductrice (2),
- une couche d'oxyde métallique (3) appliquée ou produite sur la première couche conductrice (2), et
- une couche métallique (4) appliquée sur la couche d'oxyde métallique (3),
- où le travail de sortie d'électrons de la première couche conductrice (2) est intérieur au travail de sortie d'électrons de la couche d'oxyde métallique (3), de telle manière qu'un contact ohmique est formé entre la première couche conductrice (2) et la couche d'oxyde métallique (3), et
- où un contact Schottky est également formé entre la couche d'oxyde métallique (3) et la couche métallique (4), et
- où la couche d'oxyde métallique (3) présente une structure poreuse de type éponge, et la couche métallique (4), appliquée sur la couche d'oxyde métallique (3), présente une structure poreuse correspondante,
**caractérisé en ce que**
- la première couche conductrice (200) est formée en tant que couche non autoportante sur un substrat, en particulier, un substrat en verre ou un substrat en silicium ou un substrat en céramique, et
- la première couche conductrice (2, 200) et la couche métallique poreuse (4, 400) sont reliées à une unité de commande et d'évaluation électronique, laquelle est configurée pour déterminer la température de la première couche conductrice (20, 200) en appliquant une tension latéralement à la première couche conductrice (20, 200).

2. Détecteur d'hydrogène (10, 100) selon la revendication 1, **caractérisé en ce que**
- la première couche conductrice (2) est une couche en cuivre, tantale, titane, tungstène, étain ou en un alliage de ceux-ci ou une couche TCO, en particulier une couche d'oxyde d'étain dopé au fluor,
et/ou
- la couche d'oxyde métallique (3) poreuse de type éponge présente un diamètre de pore allant de 50 nm à 1000 nm, de préférence environ 300 nm, et/ou l'épaisseur de la couche d'oxyde métallique (3) poreuse de type éponge est de 100 nm jusqu'à 5 µm, de préférence 1 µm.

3. Détecteur d'hydrogène (10, 100) selon une des revendications 1 ou 2, **caractérisé en ce que**
- la couche d'oxyde métallique (3) poreuse de type éponge est une couche en oxyde de titane, en particulier une couche en TiO₂,
et/ou
- la première couche conductrice (2) est une couche en titane et la couche d'oxyde métallique (3) poreuse de type éponge est une couche en oxyde de titane formée par anodisation de la surface de la première couche conductrice (2).
et/ou
- une couche d'oxyde métallique (3) poreuse de type éponge est déposée, à l'aide d'un processus sol-gel, sur la couche d'oxyde métallique (3) poreuse de type éponge produite par anodisation de la première couche conductrice,
et/ou
- la couche métallique (4) poreuse est une couche en platine, en palladium ou en or,
et/ou
- la couche métallique (4) poreuse présente une structure cristallitique, où les christallites présentent un diamètre allant de 5 nm à 100 nm.

4. Détecteur d'hydrogène (10, 100) selon une des revendications 1 à 3, **caractérisé en ce que**
- la première couche conductrice (20, 200) est reliée au pôle négatif de l'unité de commande et d'évaluation électronique et la couche métallique (40, 400) poreuse est reliée au pôle positif de l'unité de commande et d'évaluation électronique de sorte que l'ensemble à couches minces à diode soit exploité en direction du passage,
ou
- la première couche conductrice (20, 200) est reliée au pôle positif de l'unité de commande et d'évaluation électronique et la couche métallique (40, 400) poreuse est reliée au pôle négatif de l'unité de commande et d'évaluation électronique de sorte que l'ensemble de couches minces de diodes soit exploité en direction de blocage.

5. Détecteur d'hydrogène (10, 100) selon une des revendications 1 à 4, **caractérisé en ce que**
- le détecteur d'hydrogène (10, 100) est incorporé dans un boîtier TO (60, 600) ou fait parti d'un package grille conductrice ou LCC, où le boîtier TO (60, 600) comporte un tamis moléculaire pour la vapeur d'eau ou une membrane imperméable à l'eau et permettant la diffusion de vapeur et/ou perméable au gaz, en particulier une membrane en PTFE ou une « membrane Pall »,
ou
- le détecteur d'hydrogène est un composant CMS.

6. Procédé de production d'un détecteur d'hydrogène avec d'un ensemble à couches minces à diode (1) pour la détection d'hydrogène selon une des revendications 1 à 5, comportant les étapes de procédé suivantes :
- mise à disposition de la première couche conductrice (2),
- application de la couche d'oxyde métallique (3) sur la première couche conductrice (2) ou la production de la couche d'oxyde métallique (3) sur la première couche conductrice (2), où la couche d'oxyde métallique (3) est appliquée ou produite, à l'aide d'un procédé électrochimique ou à l'aide d'un processus sol-gel, de telle façon qu'elle forme une structure poreuse de type éponge,
- application de la couche métallique (4) sur la couche d'oxyde métallique (3), où la couche métallique (4), à l'aide d'un procédé électrochimique ou à l'aide d'un procédé de dépôt sous vide, est appliquée sur la couche d'oxyde métallique (3) de telle façon que la couche métallique (4) appliquée sur la couche d'oxyde métallique (3) présente une structure poreuse correspondant à la structure poreuse de type éponge de la couche d'oxyde métallique (3),
**caractérisé en ce que**
- la première couche conductrice (2) est formée, en tant que couche non autoportante, sur un substrat, en particulier, un substrat en verre ou un substrat en silicium ou un substrat en céramique, et
- la première couche conductrice (2, 200) et la couche métallique (4, 400) poreuse sont reliées à une unité de commande et d'évaluation électronique, laquelle est configurée pour déterminer la température de la première couche conductrice (20, 200) en appliquant une tension latéralement à la première couche conductrice (20, 200).

7. Procédé selon la revendication 6, **caractérisé en ce que**
- la première couche conductrice (2) est une couche en titane et la couche d'oxyde métallique (3) est produite par anodisation sur la couche en titane (2), où l'anodisation de la couche en titane (2) a lieu sous une tension d'anodisation allant de 90 jusqu'à 200 V, de préférence environ 120 V, où l'anodisation, en particulier, a lieu dans de l'acide sulfurique molaire de 0,5 à 14, de préférence dans de l'acide sulfurique molaire à 3,5.
ou
- la couche d'oxyde métallique (3) est appliquée par DPV sur la première couche conductrice (2).

8. Procédé selon la revendication 7, **caractérisé en ce que**
- l'on applique sur la couche d'oxyde métallique (3) poreuse de type éponge, produite par anodisation de la première couche conductrice (2), une autre couche d'oxyde métallique poreuse de type éponge par un processus sol-gel, où
- la couche d'oxyde métallique (3) poreuse de type éponge et l'autre couche d'oxyde métallique poreuse de type éponge sont chimiquement identiques.

9. Procédé selon une des revendications 6 à 8, **caractérisé en ce que** la couche métallique (4) est appliquée sur la couche d'oxyde métallique (3) poreuse de type éponge par un procédé DPV, en particulier par pulvérisation ou évaporation sous vide,.

10. Procédé selon une des revendications 6 à 9, **caractérisé en ce que** la couche métallique (4) est pourvue d'une couche de protection, en particulier d'une couche en tétraéthylorthosilicate, hexaméthyldisiloxane ou hexaméthyldisilazane.

11. Application d'un détecteur d'hydrogène (10, 100) selon une des revendications 1 à 5, en tant que détecteur d'hydrogène pour détecter des fuites, dans un véhiculé à hydrogène ou dans des réservoirs ou conduites à hydrogène.

12. Application d'un détecteur d'hydrogène (10, 100) selon une des revendications 1 à 5, en tant que détecteur d'hydrogène, dans un ensemble de détecteurs destinés à mesurer la composition de gaz, en particulier de gaz d'échappement ou gaz de ville.

13. Application d'un détecteur d'hydrogène (10, 00) selon une des revendications 1 à 5 dans un dispositif avertisseur d'incendie.

14. Application d'un détecteur d'hydrogène (10, 100) selon une des revendications 1 à 5 en tant que source d'énergie électrique, en particulier en tant que pile combustible à oxyde solide à une chambre à couche mince.

15. Application d'un détecteur d'hydrogène (10, 100) selon une des revendications 1 à 5 en tant que détecteur UV, lequel est essentiellement insensible à la lumière visible.
